# EUROPEAN PATENT SPECIFICATION

(11) **EP 3 336 198 B1**
(45) Date of publication and mention of the grant of the patent: **27.05.2020**
(21) Application number: 16204829.2
(22) Date of filing: 16.12.2016
(51) Int. Cl.: C12Q 1/689, C12Q 1/6895, C12Q 1/04, G01N 33/569

(54) **METHODS AND USES FOR DETERMINATION OF FUNGAL AND/OR BACTERIAL INFESTATION**
VERFAHREN UND VERWENDUNGEN ZUR BESTIMMUNG VON PILZ- UND/ODER BAKTERIELLEM BEFALL
PROCÉDÉS ET UTILISATIONS POUR LA DÉTERMINATION D'INFESTATION FONGIQUE ET/OU BACTÉRIENNE

(43) Date of publication of application: 20.06.2018
(73) Proprietor: HouseTest ApS, 5000 Odense C (DK)
(72) Inventor: Lindgreen, Jonas Nørrelund, 5210 Odense NV (DK); Pørneki, Ann Dorte Storm, 5492 Vissenbjerg (DK)
(74) Representative: Inspicos P/S

(56) References cited:
- JP-A- 2012 231 765
- US-A1- 2012 084 249
- ALBERT BARBERÁN ET AL: "The ecology of microscopic life in household dust", BIOLOGICAL SCIENCES, vol. 282, no. 1814, 26 August 2015 (2015-08-26), page 20151139, XP055361976, ISSN: 0962-8452, DOI: 10.1098/rspb.2015.1139
- LANGGUT DAFNA ET AL: "Resolving a historical earthquake date at Tel Yavneh (central Israel) using pollen seasonality", PALYNOLOGY, vol. 40, no. 2, 9 June 2015 (2015-06-09), pages 145-159, XP009194022,
- LIAO C M ET AL: "Use of temporal/seasonal- and size-dependent bioaerosol data to characterize the contribution of outdoor fungi to residential exposures", SCIENCE OF THE TOTAL ENVIRONMENT, ELSEVIER, AMSTERDAM, NL, vol. 347, no. 1-3, 15 July 2005 (2005-07-15), pages 78-97, XP027674253, ISSN: 0048-9697 [retrieved on 2005-07-15]
- FUMIO NAKAZAWA ET AL: "Establishing the Timing of Chemical Deposition Events on Belukha Glacier, Altai Mountains, Russia, Using Pollen Analysis", ARCTIC, ANTARCTIC, AND ALPINE RESEARCH, vol. 43, no. 1, February 2011 (2011-02), pages 66-72, XP055361792, ISSN: 1523-0430, DOI: 10.1657/1938-4246-43.1.66
- MONTALI E ET AL: "Towards a ''crime pollen calendar''-Pollen analysis on corpses throughout one year", FORENSIC SCIENCE INTERNATIONAL, ELSEVIER, AMSTERDAM, NL, vol. 163, no. 3, 22 November 2006 (2006-11-22), pages 211-223, XP027940157, ISSN: 0379-0738 [retrieved on 2006-11-22]

### SEQUENCE LISTING

<110> HouseTest ApS
<120> METHODS AND USES FOR DETERMINATION OF FUNGI AND/OR BACTERIA INFESTATION
<130> 20985EP00
<160> 12
<170> PatentIn version 3.5
<210> 1
   <211> 20
   <212> DNA
   <213> Artificial Sequence
<220>
   <223> DNA primer
<400> 1
   gccgattgtg gtgaagtgga 20
<210> 2
   <211> 18
   <212> DNA
   <213> Artificial Sequence
<220>
   <223> DNA Primer
<400> 2
   gtacgggccg ccatgaaa 18
<210> 3
   <211> 16
   <212> DNA
   <213> Artificial Sequence
<220>
   <223> DNA Primer
<400> 3
   caccgcccgt cgctac 16
<210> 4
   <211> 21
   <212> DNA
   <213> Artificial Sequence
<220>
   <223> DNA Primer
<400> 4
   taatgatcct tccgcaggtt c 21
<210> 5
   <211> 21
   <212> DNA
   <213> Artificial Sequence
<220>
   <223> DNA Primer
<400> 5
   gctttctcat tctactcttt c 21
<210> 6
   <211> 22
   <212> DNA
   <213> Artificial Sequence
<220>
   <223> DNA Primer
<400> 6
   cttttcttgt gcatcatcct ag 22
<210> 7
   <211> 22
   <212> DNA
   <213> Artificial Sequence
<220>
   <223> DNA Primer
<400> 7
   aataacaata ccgggctctt at 22
<210> 8
   <211> 15
   <212> DNA
   <213> Artificial Sequence
<220>
   <223> DNA Primer
<400> 8
   ccggtgcaca ccagg 15
<210> 9
   <211> 24
   <212> DNA
   <213> Artificial Sequence
<220>
   <223> DNA Primer
<400> 9
   caaggaactt taacgaaaga gtgc 24
<210> 10
   <211> 24
   <212> DNA
   <213> Artificial Sequence
<220>
   <223> DNA Primer
<400> 10
   gttctagaca agattcacct cccg 24
<210> 11
   <211> 19
   <212> DNA
   <213> Artificial Sequence
<220>
   <223> DNA Primer
<400> 11
   cagttgtgcg gcaaggctt 19
<210> 12
   <211> 24
   <212> DNA
   <213> Artificial Sequence
<220>
   <223> DNA Primer
<400> 12
   cattgatcaa tccaaccgat acca 24

## Description

### FIELD OF THE INVENTION

The present invention relates to methods and uses for determination of fungi and/or bacteria infestation.

### BACKGROUND OF THE INVENTION

Molds are members of a very large family called fungi. Fungi include mold, mildew, mushrooms, slimes, yeasts, smuts, and crop rusts. The fungi kingdom includes some very important organisms, in terms of both their ecological and economic roles. Molds decompose plants and other organic material, and as such play a vital role in the cycle of nature. Indoors, however, molds are very undesirable. They grow through a network of microscopic threads called hypha and disperse via airborne spores.

Molds thrive in damp, warm surroundings, but the spores can survive desiccation and re-sprout should these surroundings become humid again. Molds decompose organic matter, which are found in abundance in homes: woodwork and glue as well as wallpaper and similar paper-based materials. Consequently, molds do not always grow on bare walls, ceilings or floors, but often behind them, under wallpaper or drywalls, beneath the floorboards, under the eaves, in crawlspaces, etc. A further prerequisite for mold growth is humidity. Moisture on the surfaces of building materials may stem from water damage, but high humidity levels due to infrequent airing can also cause dampness in homes. In old, poorly insulated houses, humidity tends to condense on cold outer walls, providing ideal conditions for molds unless the place is aired regularly. Mold growths are usually key indicators of humidity issues.

According to the World Health Organization, persistent humidity and mold growths on indoor surfaces and structural elements may pose health risks and should be avoided or confined.

Physical reactions to molds vary greatly. Some people experience severe symptoms, while others in the same building are unaffected. People with allergies or asthma are more vulnerable to molds and should be particularly mindful of possible mold infestations at home and at the workplace.

Molds usually grow out of sight and are therefore difficult to locate using conventional methods. Concealed or visible, mold-infested homes pose health risks for the occupants.

Many species of mold emit a musty smell. Often, suspicions of concealed mold infestations arise as a result of various types of discomfort, such as irritation of the respiratory system and eyes, headaches, fatigue and concentration difficulties and worsening of asthma.

In order to determine the risk fungi and bacteria pose to human health, it is necessary to detect which fungi and bacteria are present and in what numbers.

In the past, the detection and quantitative measurement of fungi and bacteria in samples has been performed either by direct microscopic examination of the collected cells or by growing cells on a suitable medium and identification and enumeration of the resultant colonies. The first method is highly labor intensive and is subject to potential errors in the recognition and positive identification. The second method is both time consuming and subject to significant quantitative inaccuracy. Both methods require extensive experience on the part of the analyst.

US 6,387, 652 B1 discloses a method of identifying and quantifying specific fungi and bacteria using specific DNA sequences..

Barberán A et al. (2015), Proc. R. Soc. B 282: 20151139, discloses the analysis of fungi and bacteria in indoor settled dust and comparison to outdoor dust, thereby concluding that most indoor fungi originate from outside, whereas bacteria mostly originate from the home occupants (abstract).

State of the art methods have several drawbacks, as spores from many of the mold and bacteria species, known to grow in damp buildings, are also present in the outdoor air, from where they enter indoor environments and naturally settle in the dust. This may result in false negative or false positive samples, especially in the case where the sampled dust is old where the amount of naturally occurring mold and bacteria can reach levels comparable to those found in water damaged buildings. It is therefore difficult to distinguish between dust with high levels of mold and bacteria deriving from infested building materials, and dust samples with high levels of mold and bacteria deriving from outdoor sources.

### SUMMARY OF THE INVENTION

The invention is defined in the appended claims.

It has been found by the present inventors, that detection and quantification in a dust sample from an indoor area of seasonal pollen species such as pollen from specific plant genera present outside may be used to determine whether levels of fungi (such as mold) and/or bacteria (such as *Streptomyces spp* bacteria) also detected in the dust derives from an indoor infestation or whether the fungi and/or bacteria may derive from outdoor sources and are a result of dust collected over time.

So, in a first aspect disclosed herein is a method for determination of the origin of fungi and/or bacteria in a dust sample(s) from an indoor area, the method comprising
a) detecting qualitatively and optionally quantifying the level of one or more seasonal pollen species present in the dust sample(s),
b) detecting and quantifying the level of fungi and/or bacteria in the dust sample(s), and
c) using the identity and optionally the level of the one or more seasonal pollen species to determine whether fungi and/or bacteria present in the dust sample(s) is of indoor origin.

In a second aspect disclosed herein, is a method for antifungal and/or antibacterial treatment of an indoor area against fungal and/or bacterial infestation subsequent to a determination according to a method as disclosed herein that has established that fungi and/or bacteria in a dust sample(s) sampled from the indoor area is of indoor origin by the method.

In a third aspect disclosed herein, is a use of identity and optionally quantified level of one or more seasonal pollen species in a dust sample(s) from an indoor area to determine seasonal age of the sample(s).

In a fourth aspect disclosed herein, is a method of using identity and optionally quantified level of one or more seasonal pollen species in a dust sample(s) from an indoor area to determine whether fungi and/or bacteria present in the dust sample(s) is of indoor or outdoor origin, which method comprises the following steps:
a) detecting qualitatively and optionally quantifying the level of one or more seasonal pollen species present in the sample(s),
b) detecting and quantifying the level of fungi and/or bacteria in the sample(s), and
c) using the identity and optionally the level of the one or more seasonal pollen species to determine whether fungi and/or bacteria present in the dust sample(s) is of indoor or outdoor origin.

In a fifth aspect disclosed herein, a method for determining the seasonal age of dust sample(s) from an indoor area, which method comprises the following steps:
a) detecting qualitatively and optionally quantifying one or more seasonal pollen species present in the sample(s), and
b) using the identity and optionally the level of the one or more seasonal pollen species to determine the seasonal age of the sample(s).

In a sixth aspect disclosed herein, use of presence of one or more seasonal pollen species in a dust sample(s) from an indoor area to determine whether fungi and/or bacteria present in the dust sample(s) is of indoor or outdoor origin by:
a) detecting qualitatively and optionally quantifying the level of one or more seasonal pollen species present in the sample(s),
b) detecting and quantifying the level of fungi and/or bacteria in the sample(s), and
c) using the identity and optionally the level of the one or more seasonal pollen species to determine whether fungi and/or bacteria present in the dust sample(s) is of indoor or outdoor origin.

### DETAILED DISCLOSURE OF THE INVENTION

Disclosed herein is a method for determination of the origin of fungi and/or bacteria in a dust sample(s) from an indoor area, the method comprising
a) detecting qualitatively and optionally quantifying the level of one or more seasonal pollen species present in the dust sample(s),
b) detecting and quantifying the level of fungi and/or bacteria in the dust sample(s), and
c) using the identity and optionally the level of the one or more seasonal pollen species to determine whether fungi and/or bacteria present in the dust sample(s) is of indoor origin.

In an embodiment, step c is performed by determining whether the presence and optionally the level of seasonal pollen species detected in step a) is incompatible with the pollen and the fungi and/or bacteria having been simultaneously introduced into the dust sample from an outdoor source.

As stated above many of the fungi species such as mold, and bacteria species, known to grow in an indoor area in damp and moist buildings, are also present in the outdoor air, from where they enter indoor areas and naturally settle in the dust collected over time. This may result in false negative or false positive samples, especially in the case where the sampled dust has collected over time resulting in an amount of naturally occurring mold and bacteria comparable to those found in damp and/or water damaged infested buildings. It is therefore difficult to distinguish between dust with high levels of mold and bacteria, deriving from moist building materials and thus of indoor origin, and old dust samples with high levels of mold and bacteria deriving from outdoor sources and thus of outdoor origin.

Examples of determinations of the presence and level of seasonal pollen species detected in step a) which is incompatible with the fungi/bacteria having been simultaneously introduced into the dust sample from an outdoor source is for example shown in the examples.

### Fungi and/or bacteria

In an embodiment disclosed herein, the detection and quantifying of fungi and/or bacteria in the sample(s) in step b results in the presence of a level of one or more fungi and/or bacteria, which could be due to dust settled during a period of time or could be due to indoor infestation of the detected fungi and/or bacteria.

In the present context, the determination of whether a level of one or more fungi and/or bacteria is due to dust settled during a period of time or due to indoor infestation of the detected fungi and/or bacteria is determined by comparing with a predetermined background level of fungi and/or bacteria, and which seasonal pollen(s) is present in the dust sample. The background level to be compared with depends on the local environment, and will typically be defined as the average or median level measured in indoor environments, where there exists no confirmed infestation with fungi or bacteria for example as described under the heading "General procedures".

In an embodiment disclosed herein, fungi are selected from any fungi relevant for infestation in an indoor area such as a building. In an embodiment, fungi are building related fungi. In a further embodiment, fungi are moulds.

In an embodiment disclosed herein, fungi are selected from the group consisting of :
*Absidia spp.*
*Acremonium spp*
*Alternaria spp.*
*Aspergillus spp.*
*Aureobasidium spp.*
*Chaetomium spp.*
*Chrysonilia spp.*
*Cladosporium spp.*
*Curvularia spp.*
*Emericella spp.*
*Epicoccum spp.*
*Eurotium spp.*
*Fusarium spp.*
*Geomyces spp.*
*Geotrichum spp.*
*Gliocladium spp.*
*Gliomastix spp.*
*Memnoniella spp.*
*Mucor spp.*
*Myrothecium spp.*
*Oidiodendron spp.*
*Paecilomyces spp.*
*Penicillium spp.*
*Phialophora spp.*
*Phoma spp.*
*Rhizopus stolonifer*
*Scopulariopsis spp.*
*Sistotrema spp.*
*Stachybotrys spp.*
*Trichoderma spp.*
*Ulocladium spp.*
*Wallemia spp.*

The above mentioned fungi are all known for growing indoor in buildings.

In an embodiment disclosed herein, bacteria are selected from any bacteria relevant for infestation in buildings. In a further embodiment, the bacteria are *Streptomyces spp.*

In a further embodiment, the fungi are selected from the group consisting of:
*Acremonium strictum*
*Alternaria alternata*
*Aspergillus fumigatus*
*Aspergillus glaucus grp.*
*Aspergillus niger*
*Aspergillus versicolor*
*Chaetomium globosum*
*Cladosporium cladosporides*
*Cladosporium herbarum*
*Cladosporium sphaerospermum*
*Mucor*/*rhizop. grp*
*Penicillium*/ *Aspergillus*/*Paecilomyces spp.*
*Penicillium chrysogenum*
*Penicillium expansum*
*Rhizopus stolonifer*
*Stachybotrys chartarum*
*Tricoderma viride*
*Ulocladium chartarum*
*Wallemia sebi*
*Seasonal pollen species*

In an embodiment disclosed herein, when the level of the one or more identified seasonal pollen species in the sample(s) determined in step a is above the detection limit of the method used for detection of the identified seasonal pollen species (and thus detected qualitatively which for most purposes is sufficient) this shows that the dust has been settling for a time period starting at least since the time of year where said one or more seasonal pollen species is present in the outdoor air.

In another embodiment disclosed herein, the absence of one or more seasonal pollen species in said sample(s) determined in step a establish that the dust has been settling for a time period at the longest since the time of year where said absent one or more seasonal pollen species was present in the outdoor air.

In the present context "seasonal pollen species" means pollen, which are not perennial, i.e. the pollen are only present in trace amounts (if at all) in outdoor air samples during a period for more than 1-3 months per year in the geographical area where detection as disclosed herein of the origin of fungi/bacteria is taking place, whereas the pollen will be present in appreciable amounts in air samples during a period of time during a calendar year.

In the present context, the "absence of one or more seasonal pollen species" means that the seasonal pollen in question could not be detected qualitatively whereas "presence of one or more seasonal pollen species" means that the seasonal pollen in question was detected qualitatively above the detection limit of the method used such as by Quantitative Polymerase Chain Reaction (qPCR).

In an embodiment, seasonal pollen species is plant pollen. The presence or absence of a genus can thus reveal the seasonal age of the dust sample and make it possible to determine if fungi and/or bacteria, found in the samples, originate from indoor or outdoor sources.

In an embodiment, one or more seasonal pollen is selected from the group consisting of: *Alnus*/*corylus spp., Betula spp., Poaceae spp.* and *Artemisia spp.* These pollen are especially useful for determining the seasonal age of a dust sample from for example Northern Europe since these pollen are not present in the outside air during the whole year and are generally present in a detectable amount in indoor dust samples during the period when they are also present in the outside air.

*Alnus*/*corylus spp., Betula spp., Poaceae spp.* and *Artemisia spp* are for example very useful when detection of fungi and/or bacteria is desired in Denmark.

**Table 1 shows levels of various pollen species in the outdoor air in Denmark during a season.**

| | January | february | March | April | May | June | July | August | September | October | November | December |
|---|---|---|---|---|---|---|---|---|---|---|---|---|
| *Alnus*/*corylus spp.* | - | low | high | low | - | - | - | - | - | - | - | - |
| *Betula spp.* | - | - | low | high | high | medium | - | - | - | - | - | - |
| *Poaceae spp.* | - | - | - | - | low | high | high | medium | low | - | - | - |
| *Artemisia spp.* | - | - | - | - | - | - | high | high | low | - | - | - |

If a dust sample e.g., sampled in a Danish building in the fall, shows moderately elevated levels of mold and bacteria, but also shows the presence of all the plant pollen *Alnus*/*corylus spp., Betula spp., Poaceae spp.* and *Artemisia spp,* then it may be concluded that the dust has at least been settling since March where *Alnus*/*corylus spp.* pollen appear in the outdoor air. Based on studies of background levels of fungi and/or bacteria in the area from where the sample has been taken it is known that high levels of airborne mold and bacteria are also found in this period, and it may be concluded that the levels, found in the dust, originate from the outdoor air. If the sample had shown high mold and bacteria and *Artemisia spp.* and *Poeceae spp.* pollen, which are present in the air in late summer, but no pollen from the earlier pollen releasing genera, then it may be concluded that the dust can only be a few months old, and that the identified mold and bacteria, cannot solely be derived from the outdoor air but must also originate from indoor sources.

In an embodiment, detecting qualitatively and optionally quantifying *Alnus*/*corylus spp.* by Quantitative Polymerase Chain Reaction (qPCR) in the dust sample(s) in step a indicates that the dust has accumulated at least since the time of year where *Alnus*/*corylus spp.* was present in the outdoor air.

In an embodiment, detecting qualitatively and optionally quantifying *Betula spp.* by Quantitative Polymerase Chain Reaction (qPCR) in the dust sample(s) in step a indicates that the dust has accumulated at least since the time of year where *Betula spp.* was present in the outdoor air.

In an embodiment, detecting qualitatively and optionally quantifying *Poaceae spp.* by Quantitative Polymerase Chain Reaction (qPCR) in the dust sample(s) in step a indicates that the dust has accumulated at least since the time of year where *Poaceae spp.* was last present in the outdoor air.

In an embodiment, detecting qualitatively and optionally quantifying *Artemisia spp.* by Quantitative Polymerase Chain Reaction (qPCR) in the dust sample(s) in step a indicates that the dust has accumulated at least since the time of year where *Artemisia spp.* was last present in the outdoor air.

However, other pollen may also be chosen for use in the present methods and uses described herein depending on the area in which detection of fungi and/or bacteria are desired. Examples of other pollen are *Quercus spp., Ulmus spp., Salix spp., and Rumex sp..*

### Sampling

A sample of dust from an indoor area may be collected by any suitable method known to the skilled person. In an embodiment, a swab comprising a rod and a tip for example a cotton swab or a FLOQSwab may be used. The dust may be collected by scraping a swab along a 15 cm² horizontal, smooth surface area at least 50 cm above floor height.

In an embodiment disclosed herein, more than one sample may be collected from the same indoor area. This has the advantage of better being able to locate the microbial source. The detection and/or quantification of pollen in step a and the fungi/bacteria in step b may also suitably be determined in the same sample, such that step a and step b is performed as one step. This has the advantage of simplifying the method, and to make sure that pollen and fungi/bacteria is measured in dust with the same age.

In an embodiment, the detection and/or quantification of one or more seasonal pollen species in step a and the detection and quantification of fungi and/or bacteria in step b is performed on the same sample, optionally in the same operation.

### Detecting and quantifying the level of fungi and/or bacteria in the dust sample(s)

Fungi and bacteria may be detected (or identified) and quantified by using for example a nucleotide sequence specific to the particular species or, in the case of some fungi, of a group of species. There exist many methods known to the skilled person including using SYBR-green based Quantitative Polymerase Chain Reaction (qPCR), probe-based Quantitative Polymerase Chain Reaction (qPCR) or other methods of detection and quantification including hybridization or conventional PCR could be used with these sequences.

The qPCR methods described in US patent 6,387,652 may be generally applied in the methods and uses described herein for detection and quantifying the level of the one or more fungi and/or bacteria. These qPCR methods may also be used to detect and quantify the seasonal pollen species(s).

Genomic DNA may be extracted using standard methods, e.g., the glass bead milling and glass milk adsorption method or any similar procedure of extracting genomic DNA.

For each targeted fungus or bacterium, the appropriate forward primer and reverse primer are to be obtained.

For each target species or group of species, calibrator samples with a known numbers of conidia or cells are used to create calibration curves.

In one embodiment, the means for detecting and quantifying the specific fungi or bacteria in the sample in step b is by using a DNA hybridizing method, such as Quantitative Polymerase Chain Reaction (qPCR) r microarray comprising the steps:
1) extracting and recovering DNA from the organism in the sample
2) hybridizing and amplifying the DNA sequences such that the specific fungi and/or bacterium can be identified and quantified without interference from DNA from other sources.

In an embodiment, detecting and quantifying the level of one or more fungi and/or bacteria in step b is performed by using a nucleotide sequence specific to each species or group of species of fungi and/or bacteria.

In an embodiment, forward and reverse primers for specific fungi and for "universal fungal" are selected from the group described in US 6,387, 652 B1 in table 1"List of Fungal Primers and Probes" SEQ ID Nos: 1-218, columns 3-18.

Useful fungal primer sequences for Absidia | Acremonium | Alternaria | Aspergillus | Aureobasidium | Candida | Chaetomium | Cladosporium | Emericella | Eurotium (also named Asp glaucus) | Epicoccum | Fusarium | Geotrichum | Memnoniella | Mucor | Myrothecium | Paecilomyces | Penicillium | Rhizomucor | Rhizopus | Scopulariopsis | Stachybotrys | Trichoderma | Ulocladium | Wallemia | Universial Penicillium, Aspergillus, Paecilomyces | may also be found in the document entitled "Microbiological and Chemical Exposure Assessment" from EPA (https://irp-cdn.multiscreensite.com/c4e267ab/files/uploaded/gCQnkBNWQuSD96fPIikY_EPA_Technology %20for%20Mold%20Identification%20and%20Enumeration.pdf). Reference is also made to the article "Evaluation of quantitative PCR and culture methods for detection of house dust fungi and streptomycetes in relation to moisture damage of the house" by Lignell et al. in Letters in Applied Microbiology ISSN 0266-8254; 2008, National Public Health Institute, Department of Environmental Health, Kuopio, Finland.

In a further embodiment, an example of a forward and reverse primer for *Streptomyces spp.* is listed below:

| **Target** | **Forward primer** | **Reverse primer** |
|---|---|---|
| *Streptomyces spp.* | GCC GAT TGT GGT GAA GTG GA SEQ ID No: 1 | GTA CGG GCC GCC ATG AAA SEQ ID No: 2 |

In the present context, "universal fungi" means a primer set designed to detect all members of the fungi kingdom.

In a further embodiment, an example of a forward and reverse primer for "universal fungi" is listed below:

| **Target** | **Forward primer** | **Reverse primer** |
|---|---|---|
| Universal fungi | CACCGCCCGTCGCTAC SEQ ID No: 3 | TAATGATCCTTCCGCAGGTTC SEQ ID No: 4 |

In an embodiment herein, fungi and/or bacteria to be positively present for the purposes of the herein disclosed methods and uses should be detected and quantified in a level above the detection limit as for example determined by the method described in Examples 1-3.

### Detecting and optionally quantifying the level of one or more seasonal pollen species present in the dust sample(s)

Pollen may be detected qualitatively (or identified) and/or quantified by any method known to the skilled person. In an embodiment disclosed herein a nucleotide sequence specific to the particular species or a group of species of pollens is used. The same methods known to the skilled person mentioned above in regards to fungi and/or bacteria may also be used for detection qualitatively and/or quantification of pollen.

In an embodiment, the means for detecting qualitatively and optionally quantifying the specific seasonal pollen species in the sample in step a is by using a DNA hybridizing method, such as Quantitative Polymerase Chain Reaction (qPCR) or microarray comprising the steps:
1) extracting and recovering DNA from seasonal pollen species in the sample
2) hybridizing and amplifying the DNA sequences such that the specific seasonal pollen species can be identified and optionally quantified without interference from DNA in the sample belonging to other sources.

In an embodiment, detecting qualitatively and optionally quantifying the level of the one or more seasonal pollen species in step a is performed by using a nucleotide sequence specific to each species or group of species of pollen.

In an embodiment, primers for seasonal pollen species are selected from the group consisting of:

| **Genera** | **Forward primer** | **Reverse primer** |
|---|---|---|
| *Poeceae spp.* | GCT TTC TCA TTC TAC TCT TTC SEQ ID No: 5 | CTT TTC TTG TGC ATC ATC CTA G SEQ ID No: 6 |
| *Alnus*/*corylus spp* | AAT AAC AAT ACC GGG CTC TTA T SEQ ID No: 7 | CCG GTG CAC ACC AGG SEQ ID No: 8 |
| *Betula spp.* | CAA GGA ACT TTA ACG AAA GAG TGC SEQ ID No: 9 | GTT CTA GAC AAG ATT CAC CTC CCG SEQ ID No: 10 |
| *Artemisia spp.* | CAG TTG TGC GGC AAG GCT T SEQ ID No: 11 | CAT TGA TCA ATC CAA CCG ATA CCA SEQ ID No: 12 |

In order for seasonal pollen to be positively present for the purposes of the herein disclosed methods and uses they should be detected qualitatively above the detection limit for the method used as for example by qPCR as in Example 1.

In some embodiments, it might be preferred to also quantify the amount of the one or more seasonal pollen for example in order to establish how far from the detection limit the amount is.

### Determination of the origin of fungi and/or bacteria in a dust sample(s) from an indoor area

In an embodiment disclosed herein, it may be concluded whether the quantified level of fungi and/or bacteria is due to dust settled during a period of time or is due to indoor infestation of the detected fungi and/or bacteria based on the quantified level of fungi and/or bacteria in the dust sample(s) in step b compared with background levels and the period of time the dust has been settled as determined by using the identity and the level of one or more seasonal pollen species or the absence of one or more seasonal pollen species.

As mentioned above pollen from the plant genera *Alnus*/*corylus spp., Betula spp.,* and *Artemisia spp.* are only found in short and specific time intervals during a year, the presence or absence of a genus in a dust sample above the detection limit may therefore reveal the seasonal age of the dust sample and thus make it possible to determine if detected fungi and/or bacteria, found in the samples, originate from indoor or outdoor sources.

If it is found that the detected fungi and/or bacteria are of indoor origin, the method disclosed herein comprises the step of antifungal and/or antibacterial treatment of the indoor area.

Methods of antifungal and/or antibacterial treatment of an indoor area are known to the skilled person and may for example be performed as described in EPA: Mold Remediation in Schools and Commercial Buildings Guide https://www.epa.gov/mold/mold-remediation-schools-and-commercial-buildings-guide.

In a further embodiment disclosed herein, is method for antifungal and/or antibacterial treatment of an indoor area against fungal and/or bacterial infestation subsequent to a determination according to a method disclosed herein that has established that fungi and/or bacteria in a dust sample(s) sampled from the indoor area is of indoor origin by the method.

In a further embodiment disclosed herein, the identity and optionally quantified level of one or more seasonal pollen species in a dust sample(s) from an indoor area is used to determine the seasonal age of the sample(s).

In a further embodiment disclosed herein is a method of using identity and optionally quantified level of one or more seasonal pollen species in a dust sample(s) from an indoor area to determine whether fungi and/or bacteria present in the dust sample(s) is of indoor or outdoor origin, which method comprises the following steps:
a) detecting qualitatively and optionally quantifying the level of one or more seasonal pollen species present in the sample(s),
b) detecting and quantifying the level of fungi and/or bacteria in the sample(s), and
c) using the identity and optionally the level of the one or more seasonal pollen species to determine whether fungi and/or bacteria present in the dust sample(s) is of indoor or outdoor origin.

In a further embodiment disclosed herein is a method for determining the seasonal age of dust sample(s) from an indoor area, which method comprises the following steps:
a) detecting qualitatively and optionally quantifying one or more seasonal pollen species is present in the sample(s), and
b) using the identity and optionally the level of the one or more seasonal pollen species to determine the seasonal age of the sample(s).

In a further embodiment disclosed herein, presence of one or more seasonal pollen species in a dust sample(s) from an indoor area is used to determine whether fungi and/or bacteria present in the dust sample(s) is of indoor or outdoor origin by:
a) detecting qualitatively and optionally quantifying the level one or more seasonal pollen species present in the sample(s),
b) detecting and quantifying the level of fungi and/or bacteria in the sample(s), and
c) using the identity and optionally the level of the one or more seasonal pollen species to determine whether fungi and/or bacteria present in the dust sample(s) is of indoor or outdoor origin.

Further embodiments disclosed herein:
Embodiment 1. A method for determination of the origin of fungi and/or bacteria in a dust sample(s) from an indoor area, the method comprising
a) detecting qualitatively and optionally quantifying the level of one or more seasonal pollen species present in the dust sample(s),
b) detecting and quantifying the level of fungi and/or bacteria in the dust sample(s), and
c) using the identity and optionally the level of the one or more seasonal pollen species to determine whether fungi and/or bacteria present in the dust sample(s) is of indoor origin.

Embodiment 2. The method according to embodiment 1, wherein step c is performed by determining whether the presence and optionally the level of seasonal pollen species detected in step a) is incompatible with the pollen and the fungi and/or bacteria having been simultaneously introduced into the dust sample from an outdoor source.
Embodiment 3. The method according to any one of embodiments 1-2, wherein the detection and quantifying of fungi and/or bacteria in the sample(s) in step b results in the presence of a level of one or more fungi and/or bacteria, which could be due to dust settled during a period of time or could be due to indoor infestation of the detected fungi and/or bacteria.
Embodiment 4. The method according to any one of embodiments 1-3, wherein the detection of seasonal pollen species in said sample(s) determined in step a establish that the dust has been settling for a time period starting at least since the time of year where said one or more seasonal pollen species is present in the outdoor air.
Embodiment 5. The method according to any one of embodiments 1-4, wherein the absence of one or more seasonal pollen species in said sample(s) determined in step a establish that the dust has been settling for a time period at the longest since the time of year where said absent one or more seasonal pollen species was present in the outdoor air.
Embodiment 6. The method according to any one of embodiments 1-5, wherein based on the quantified level of fungi and/or bacteria in the dust sample(s) in step b and background levels for fungi and/or bacteria compared with the period of time the dust has been settled as determined according to embodiment 4 and/or 5 conclude whether the level of fungi and/or bacteria is due to dust settled during a period of time or is due to indoor infestation of the detected fungi and/or bacteria.
Embodiment 7. The method according to any one of embodiments 1-6, wherein the one or more seasonal pollen species is selected from the group consisting of:
*Alnus*/*corylus spp., Betula spp., Poaceae spp.* and *Artemisia spp.*
Embodiment 8. The method according to any one of embodiments 1-7, wherein detecting qualitatively and optionally quantifying the one or more seasonal pollen species in step a is by using a nucleotide sequence specific to each species or group of species of pollen.
Embodiment 9. The method according to any one of embodiments 1-8, wherein detecting and quantifying the level of one or more fungi and/or bacteria in step b is by using a nucleotide sequence specific to each species or group of species of fungi and/or bacteria.
Embodiment 10. The method according to any one of embodiments 1-9, wherein detecting qualitatively and optionally quantifying *Alnus*/*corylus spp.* by Quantitative Polymerase Chain Reaction (qPCR) in the dust sample(s) in step a indicates that the dust has accumulated at least since the time of year where *Alnus*/*corylus spp.* was present in the outdoor air.
Embodiment 11. The method according to any one of embodiments 1-10, wherein detecting qualitatively and optionally quantifying *Betula spp.* by Quantitative Polymerase Chain Reaction (qPCR) in the dust sample(s) in step a indicates that the dust has accumulated at least since the time of year where *Betula spp.* was present in the outdoor air.
Embodiment 12. The method according to any one of embodiments 1-11, wherein detecting qualitatively and optionally quantifying *Poaceae spp.* by Quantitative Polymerase Chain Reaction (qPCR) in the dust sample(s) in step a indicates that the dust has accumulated at least since the time of year where *Poaceae spp.* was last present in the outdoor air.
Embodiment 13. The method according to any one of embodiments 1-12, wherein detecting qualitatively and optionally quantifying *Artemisia spp.* by Quantitative Polymerase Chain Reaction (qPCR) in the dust sample(s) in step a indicates that the dust has accumulated at least since the time of year where *Artemisia spp.* was last present in the outdoor air.
Embodiment 14. The method according to any one of embodiments 1-13, wherein the detection and optionally quantification of one or more seasonal pollen species in step a and the detection and quantification of the fungi and/or bacteria in step b is performed on the same sample, optionally in the same operation.
Embodiment 15. The method according to any one of embodiments 1-14, wherein the fungi and/or bacteria are selected from the group consisting of:
*Absidia spp.*
*Acremonium spp*
*Alternaria spp.*
*Aspergillus spp.*
*Aureobasidium spp.*
*Chaetomium spp.*
*Chrysonilia spp.*
*Cladosporium spp.*
*Curvularia spp.*
*Emericella spp.*
*Epicoccum spp.*
*Eurotium spp.*
*Fusarium spp.*
*Geomyces spp.*
*Geotrichum spp.*
*Gliocladium spp.*
*Gliomastix spp.*
*Memnoniella spp.*
*Mucor spp.*
*Myrothecium spp.*
*Oidiodendron spp.*
*Paecilomyces spp.*
*Penicillium spp.*
*Phialophora spp.*
*Phoma spp.*
*Rhizopus stolonifer*
*Scopulariopsis spp.*
*Sistotrema spp.*
*Stachybotrys spp.*
*Streptomyces spp.*
*Trichoderma spp.*
*Ulocladium spp.*
*Wallemia spp.*

Embodiment 16. The method according to embodiment 15, wherein the fungi and/or bacteria are selected from the group consisting of:
*Acremonium strictum*
*Alternaria alternata*
*Aspergillus fumigatus*
*Aspergillus glaucus grp.*
*Aspergillus niger*
*Aspergillus versicolor*
*Chaetomium globosum*
*Cladosporium cladosporides*
*Cladosporium herbarum*
*Cladosporium sphaerospermum*
*Mucor*/*rhizop. grp*
*Penicillium*/ *Aspergillus*/*Paecilomyces spp.*
*Penicillium chrysogenum*
*Penicillium expansum*
*Rhizopus stolonifer*
*Stachybotrys chartarum*
*Streptomyces spp.*
*Tricoderma viride*
*Ulocladium chartarum*
*Wallemia sebi*

Embodiment 17. The method according to any one of the embodiments 1-16, wherein the means for detecting and quantifying the specific fungi or bacteria in the sample in step b is by using a DNA hybridizing method, such as Quantitative Polymerase Chain Reaction (qPCR) or microarray comprising the steps:
1) extracting and recovering DNA from the organism in the sample,
2) hybridizing and amplifying the DNA sequences such that the specific fungi and/or bacterium can be identified and quantified without interference from other sources.

Embodiment 18. The method according to any one of the embodiments 1-19, wherein the means for detecting and optionally quantifying the specific seasonal pollen species in step a in the sample is by using a DNA hybridizing method, such as Quantitative Polymerase Chain Reaction (qPCR) or microarray comprising the steps:
1) extracting and recovering DNA from pollen in the sample,
2) hybridizing and amplifying the DNA sequences such that the specific pollen can be identified and optionally quantified without interference from other sources.

Embodiment 19. The method according to embodiment 18, wherein the primers for the seasonal pollen species are selected from the group consisting of:

| **Genera** | **Forward primer** | **Reverse primer** |
|---|---|---|
| *Poeceae spp.* | GCT TTC TCA TTC TAC TCT TTC SEQ ID No: 5 | CTT TTC TTG TGC ATC ATC CTA G SEQ ID No: 6 |
| *Alnus*/*corylus spp* | AAT AAC AAT ACC GGG CTC TTA T SEQ ID No: 7 | CCG GTG CAC ACC AGG SEQ ID No: 8 |
| *Betula spp.* | CAA GGA ACT TTA ACG AAA GAG TGC SEQ ID No: 9 | GTT CTA GAC AAG ATT CAC CTC CCG SEQ ID No: 10 |
| *Artemisia spp.* | CAG TTG TGC GGC AAG GCT T SEQ ID No: 11 | CAT TGA TCA ATC CAA CCG ATA CCA SEQ ID No: 12 |

Embodiment 20. The method according to any one of embodiments 1-19 further comprising the step of antifungal and/or antibacterial treatment of the indoor area if the origin of fungi and/or bacteria in the dust sample has been determined to be of indoor origin.
Embodiment 21. A method for antifungal and/or antibacterial treatment of an indoor area against fungal and/or bacterial infestation subsequent to a determination according to any one of embodiments 1-20 that has established that fungi and/or bacteria in a dust sample(s) sampled from the indoor area is of indoor origin by the method.
Embodiment 22. Use of identity and optionally quantified level of one or more seasonal pollen species in a dust sample(s) from an indoor area to determine seasonal age of the sample(s).
Embodiment 23. The use according to embodiment 22 further defined as in any one of embodiments 1-20.
Embodiment 24. A method of using identity and optionally quantified level of one or more seasonal pollen species in a dust sample(s) from an indoor area to determine whether fungi and/or bacteria present in the dust sample(s) is of indoor or outdoor origin, which method comprises the following steps:
a) detecting qualitatively and optionally quantifying the level of one or more seasonal pollen species present in the sample(s),
b) detecting and quantifying the level of fungi and/or bacteria in the sample(s), and
c) using the identity and optionally the level of the one or more seasonal pollen species to determine whether fungi and/or bacteria present in the dust sample(s) is of indoor or outdoor origin.

Embodiment 25. The method according to embodiment 24 further defined as in any one of embodiments 1-20.
Embodiment 26. A method for determining the seasonal age of dust sample(s) from an indoor area, which method comprises the following steps:
a) detecting qualitatively and optionally quantifying one or more seasonal pollen species present in the sample(s), and
b) using the identity and optionally the level of the one or more seasonal pollen species to determine the seasonal age of the sample(s).

Embodiment 27. The method according to embodiment 26 further defined as in any one of embodiments 1-20.
Embodiment 28. Use of presence of one or more seasonal pollen species in a dust sample(s) from an indoor area to determine whether fungi and/or bacteria present in the dust sample(s) is of indoor or outdoor origin by:
a) detecting qualitatively and optionally quantifying the level of one or more seasonal pollen species present in the sample(s),
b) detecting and quantifying the level of fungi and/or bacteria in the sample(s), and
c) using the identity and optionally the level of the one or more seasonal pollen species to determine whether fungi and/or bacteria present in the dust sample(s) is of indoor or outdoor origin.

Embodiment 29. The use according to embodiment 28 further defined as in any one of embodiments 1-20.

### GENERAL PROCEDURES

*Determination of background level of bacteria and*/*or fungi during a year and determining in which seasonal intervals during a year the chosen seasonal pollen species are present.*

The background level of specific bacteria or fungi during a year depends on the local environment, and will typically be determined as the average or median level measured in indoor environments, where there is no confirmed infestation with fungi or bacteria. This determination may be performed by quantifying the levels for all relevant selected species/groups of fungi in fixed time intervals (e.g. every 14 days) in the same buildings during a year, while using the same sample site every time so the precise age of the dust is known and then quantify the levels of the chosen fungi and/or bacteria in the samples by for example qPCR. The number of buildings may for example be at least 50 different buildings, which buildings have all been thoroughly examined to make sure that there is no indoor growth of fungi or bacteria.

The levels of relevant seasonal pollen species such as plant pollen known to be present in the outdoor air in the given area during a year may be determined by a literature search, by testing of air samples during a year, by testing of dust samples from buildings during a year or using public available pollen counts.

In an embodiment, determination in which intervals during a season the chosen pollen is naturally occurring may be performed by sampling house dust in fixed time intervals (e.g. every 14 days) in the same buildings, while using the same sample site every time so the precise age of the dust is known and then quantify the levels of the chosen pollen in the samples by qPCR.

Any sample showing fungi or bacteria in higher amounts than the background levels for a given age of the dust as determined by analyzing pollen levels, will indicate indoor growth of fungi and/or bacteria.

### EXAMPLE 1

Dust was sampled using a FLOQswab by scraping it along a 15 cm² horizontal, smooth surface area at least 50 cm above floor height in April 2015 in a Danish building (sample 1). The dust was expected to be between one and twelve months old.

Genomic DNA was extracted by lysing cells by glass bead milling (0.5 g acid washed glass beads, 300 µl digestion buffer (Thermo Scientific for 2,000 RPM in 20 min) in 1.5 ml Eppendorf tubes. The DNA was purified using a GeneJET Genomic DNA Purification Kit - Thermo Scientific as described in the kit manual.

Reactions was prepared in 96- or 384 welled qPCR plates by addition of the following components: 10 µl of RealQ PCR 2x Master Mix with Green Dye, 5 µl DNA template and 5 µl of a forward and reverse mixture of primers at the optimal concentration determined for each specific primer set.

The qPCR assay was performed on a real-time system CFX 96™ Real Time system, Bio-Rad under the following conditions: 5 min denaturation at 96 °C, 43 cycles of 96 °C for 15 s, 66 °C for 30 °C and 72 °C for 30 s followed by a melting curve analysis. All reactions including positive and negative controls were run at least in triplicate.

For each target species calibrator samples with various known number of pollen was prepared and used as standards.

To quantify the mould levels, qPCR using fungi and/or bacteria specific primers were performed.

The results showed the following spore levels:

**Table 2: Mold and bacteria in sample 1**

| | | | |
|---|---|---|---|
| *Universal fungi:* | 35.037 | *Cladosporium sphae.* | 43 |
| *Acremonium strictum* | 0 | *Mucor*/*Rhizopus Grp* | 66 |
| *Alternaria alternata* | 2 | *Pen.*/*Asp.*/*Pae. grp* | 1.359 |
| *Aspergillus fumigatus* | 154 | *Penicillum Chrysogenum* | 3 |
| *Aspergillus glaucus grp.* | 94 | *Rhizopus stolonifer* | 0 |
| *Aspergillus niger* | 1 | *Stachybotrys chartarum* | 0 |
| *Aspergillus versicolor* | 92 | *Streptomyces spp.* | 420 |
| *Chaetomium globosum* | 0 | *Trichoderma viride* | 0 |
| *Cladosporium dado.* | 1.521 | *Ulocladium chartarum* | 0 |
| *Cladosporium herbarum* | 1.754 | *Wallemia sebi* | 49 |

When comparing with known background levels for the area, the levels of *Apsergillus fumigatus, Aspergillus glaucus grp.* and *Pen.*/*Asp.*/*Pae. grp* were higher than average for dust collected in a building without mold infestation unless the dust is roughly six months or older.

Thus, there are two possible explanations for the observed levels of mold:
Scenario 1: the dust sample is 6 months or older, and the mold spores originate from the outdoor air, and are not an indication of mold problem in the building.
Scenario 2: the dust is younger, and the building from where the dust is sampled has an infestation of the three species/groups of mold.

To identify the correct scenario, pollen levels from the above mentioned species are measured by qPCR in the same sample 1 using the following primers:

**Table 3: Forward and reverse primers**

| **Genera** | **Forward primer** | **Reverse primer** |
|---|---|---|
| *Poeceae spp.* | GCT TTC TCA TTC TAC TCT TTC SEQ ID No: 5 | CTT TTC TTG TGC ATC ATC CTA G SEQ ID No: 6 |
| *Alnus*/*corylus spp* | AAT AAC AAT ACC GGG CTC TTA T SEQ ID No: 7 | CCG GTG CAC ACC AGG SEQ ID No: 8 |
| *Betula spp.* | CAA GGA ACT TTA ACG AAA GAG TGC SEQ ID No: 9 | GTT CTA GAC AAG ATT CAC CTC CCG SEQ ID No: 10 |
| *Artemisia spp.* | CAG TTG TGC GGC AAG GCT T SEQ ID No: 11 | CAT TGA TCA ATC CAA CCG ATA CCA SEQ ID No: 12 |

The analysis gave the following Cq-values:

**Table 4: Pollen in sample 1**

| | |
|---|---|
| *Alnus*/*corylus spp.* | 32.08 |
| *Betula spp.* | 31.46 |
| *Artemisia spp* | 34.76 |
| *Poaceae spp.* | 32.72 |

The Cq values from table 4 were compared to negative control Cq-values for each primer set which was previously obtained:

**Table 5: Negative control Cq-values for pollen primers**

| | |
|---|---|
| *Alnus*/*corylus spp.* | 34 |
| *Betula spp.* | 36 |
| *Artemisia spp.* | 36 |
| *Poaceae spp.* | 34 |

Cq-values below negative control values show that the specific pollen species have been present in the air, at some point during which the dust has been settling. In example 1 comparing the pollen levels with the negative control values show that the dust has been settling when *Alnus*/*corylus spp., Betula spp., Artemisia spp.* and *Poaceae spp.* were present in the air.

Knowing that dust was sampled in April 2015, and by comparing the pollen levels with table 1 it may be inferred that the dust has been settling since the former spring/early summer, and thus the age of the dust can fully explain the measured mould levels thereby supporting scenario 1.

### EXAMPLE 2

A dust sample (sample 2) of unknown age was sampled August 2015 from a Danish building, and analysed by qPCR for mold, bacteria and pollen as described above. Results are listed in table 6 and 7.

**Table 6: Mold and bacteria in sample 2**

| | | | |
|---|---|---|---|
| *Universal fungi:* | 77.384 | *Cladosporium sphae.* | 349 |
| *Acremonium strictum* | 0 | *Mucor*/*Rhizopus Grp* | 34 |
| *Alternaria alternata* | 6 | *Pen.*/*Asp.*/*Pae. grp* | 7.518 |
| *Aspergillus fumigatus* | 53 | *Penicillum Chrysogenum* | 68 |
| *Aspergillus glaucus grp.* | 52 | *Rhizopus stolonifer* | 0 |
| *Aspergillus niger* | 49 | *Stachybotrys chartarum* | 15 |
| *Aspergillus versicolor* | 814 | *Streptomyces spp.* | 439 |
| *Chaetomium globosum* | 0 | *Trichoderma viride* | 0 |
| *Cladosporium dado.* | 2.977 | *Ulocladium chartarum* | 0 |
| *Cladosporium herbarum* | 4.819 | *Wallemia sebi* | 378 |

**Table 7: Pollen in sample 2**

| | |
|---|---|
| *Alnus*/*corylus spp.* | 34.35 |
| *Betula spp.* | 31.48 |
| *Artemisia spp* | 35.46 |
| *Poaceae spp.* | 31.32 |

When comparing with known background levels for the area, the levels of *Apsergillus fumigatus, Aspergillus glaucus grp, Aspergillus niger, Pen.*/*Asp.*/*Pae. grp* and *Wallemia Sebi* were higher than average for dust collected in a building without mold infestation unless the dust is roughly 8-10 months or older.

As in example 1 there are to possible scenarios that can explain the observed mold levels:
Scenario 1: the dust sample is roughly 8-10 months or older, and the mold spores originate from the outdoor air, and are not an indication of mold problem in the building.
Scenario 2: the dust is younger, and the building from where the dust is sampled has an infestation of the five species/groups of mold.

The absence of Alnus/corylus spp and presence of the other pollen species show that the dust has been settling since April/May, and the relative young age of the dust can therefore not explain the high levels of mold alone, thereby supporting scenario 2.

### EXAMPLE 3

A dust sample (sample 3) of unknown age was taken in November 2016 from a Danish building, and analysed by qPCR for mold, bacteria and pollen. Results are listed in table 8 and 9.

**Table 8: Mold and bacteria in sample 2**

| | | | |
|---|---|---|---|
| Universal fungi: | 243.249 | *Cladosporium sphaerospernum* | 4.558 |
| *Acremonium strictum* | 346 | *Mucor*/*Rhizopus Grp* | 83 |
| *Alternaria alternata* | 86 | *Pen.*/*Asp.*/*Pae. grp* | 10.841 |
| *Aspergillus fumigatus* | 300 | *Penicillum Chrysogenum* | 60 |
| *Aspergillus glaucus grp.* | 40 | *Rhizopus stolonifer* | 3 |
| *Aspergillus niger* | 35 | *Stachybotrys chartarum* | 3 |
| *Aspergillus versicolor* | 1.500 | *Streptomyces spp.* | 520 |
| *Chaetomium globosum* | 2 | *Trichoderma viride* | 0 |
| *Cladosporium cladosporides* | 14.774 | *Ulocladium chartarum* | 4 |
| *Cladosporium herbarum* | 62.096 | *Wallemia sebi* | 293 |

**Table 9: Pollen in sample 2**

| | |
|---|---|
| Alnus/corylus spp. | N/A (no detection) |
| Betula spp. | 30,33 |
| Artemisia spp | 30,92 |
| Poaceae spp. | 32,37 |

When comparing with known background levels for the area, the levels of *Acremonium strictum, Apsergillus fumigatus, Aspergillus versicolor, Cladosporium cladosporides, Cladosporium herbarum, Pen.*/*Asp.*/*Pae.* grp and *Wallemia Sebi* were much higher than average for dust collected in a building without mold infestation.

As in example 1 and 2 there are to possible scenarios that can explain the observed mold levels:
Scenario 1: Based on the knowledge on mold background levels in the area the dust sample is at least 1 year old, and the mold spores originate from the outdoor air, and are not an indication of mold problem in the building.
Scenario 2: the dust is younger, and the building from where the dust is sampled has an infestation of the five species/groups of mold.

The absence of Alnus/corylus spp and presence of the other pollen species show that the dust has been settling since April/May, and the relative young age of the dust can therefore not explain the high levels of mold alone, thereby supporting scenario 2.

## Claims

1. A method for determination of the origin of fungi and/or bacteria in a dust sample(s) from an indoor area, the method comprising
a) detecting qualitatively one or more seasonal pollen species present in the dust sample(s),
b) detecting and quantifying the level of fungi and/or bacteria in the dust sample(s), and
c) using the identity of the one or more seasonal pollen species to determine whether fungi and/or bacteria present in the dust sample(s) is of indoor origin by concluding based on the quantified level of fungi and/or bacteria in the dust sample(s) in step b and background levels for fungi and/or bacteria compared with the period of time the dust has been settled as determined according to step a whether the level of fungi and/or bacteria is due to dust settled during a period of time or is due to indoor infestation of the detected fungi and/or bacteria,
wherein the absence of one or more seasonal pollen species in said sample(s) determined in step a establish that the dust has been settling for a time period at the longest since the time of year where said absent one or more seasonal pollen species was present in the outdoor air and/or wherein the detection of seasonal pollen species in said sample(s) determined in step a establish that the dust has been settling for a time period starting at least since the time of year where said one or more seasonal pollen species is present in the outdoor air.

2. The method according to claim 1, wherein step c is performed by determining whether the presence and optionally the level of seasonal pollen species detected in step a) is incompatible with the pollen and the fungi and/or bacteria having been simultaneously introduced into the dust sample from an outdoor source.

3. The method according to any one of claims 1-2, wherein the level of the one or more seasonal pollen is quantified in step a.

4. The method according to any one of claims 1-3, wherein the qualitatively detection and quantifying of the level of the one or more seasonal pollen species in step a is performed by using a nucleotide sequence specific to each species or group of species of pollen.

5. The method according to any one of claims 1-4, wherein the one or more seasonal pollen species is selected from the group consisting of:
*Alnus*/*corylus spp., Betula spp., Poaceae spp.* and *Artemisia spp.*

6. The method according to any one of claims 1-5, wherein the detection and optionally quantification of one or more seasonal pollen species in step a and the detection and quantification of the fungi and/or bacteria in step b is performed on the same sample and in the same operation.

7. The method according to any one of claims 1-6, wherein the fungi and/or bacteria are selected from the group consisting of:
*Absidia spp.*
*Acremonium spp*
*Alternaria spp.*
*Aspergillus spp.*
*Aureobasidium spp.*
*Chaetomium spp.*
*Chrysonilia spp.*
*Cladosporium spp.*
*Curvularia spp.*
*Emericella spp.*
*Epicoccum spp.*
*Eurotium spp.*
*Fusarium spp.*
*Geomyces spp.*
*Geotrichum spp.*
*Gliocladium spp.*
*Gliomastix spp.*
*Memnoniella spp.*
*Mucor spp.*
*Myrothecium spp.*
*Oidiodendron spp.*
*Paecilomyces spp.*
*Penicillium spp.*
*Phialophora spp.*
*Phoma spp.*
*Rhizopus stolonifer*
*Scopulariopsis spp.*
*Sistotrema spp.*
*Stachybotrys spp.*
*Streptomyces spp.*
*Trichoderma spp.*
*Ulocladium spp.*
*Wallemia spp.*

8. The method according to claim 7, wherein the fungi and/or bacteria are selected from the group consisting of:
*Acremonium strictum*
*Alternaria alternata*
*Aspergillus fumigatus*
*Aspergillus glaucus grp.*
*Aspergillus niger*
*Aspergillus versicolor*
*Chaetomium globosum*
*Cladosporium cladosporides*
*Cladosporium herbarum*
*Cladosporium sphaerospermum*
*Mucor*/*rhizop. grp*
*Penicillium*/*Aspergillus*/*Paecilomyces spp.*
*Penicillium chrysogenum*
*Penicillium expansum*
*Rhizopus stolonifer*
*Stachybotrys chartarum*
*Streptomyces spp.*
*Tricoderma viride*
*Ulocladium chartarum*
*Wallemia sebi*

9. The method according to any one of claims 1-8 further comprising the step of antifungal and/or antibacterial treatment of the indoor area if the origin of fungi and/or bacteria in the dust sample is of indoor origin.

10. Use of qualitative detection of seasonal pollen in determination of the origin of fungi and/or bacteria from an indoor dust sample by a method as defined in any one of claims 1-9.

## Patentansprüche

1. Verfahren für die Bestimmung des Ursprungs von Pilzen und/oder Bakterien in einer Staubprobe/in Staubproben aus einem Innenbereich, wobei das Verfahren Folgendes umfasst
a) qualitatives Feststellen einer oder mehrerer jahreszeitlicher Pollenspezies, die in der/den Staubprobe(n) vorliegen,
b) Feststellen und Quantifizieren des Niveaus an Pilzen und/oder Bakterien in der/den Staubprobe(n) und
c) Verwenden der Identität der einen oder mehreren jahreszeitlichen Pollenspezies zum Bestimmen, ob Pilze und/oder Bakterien, die in der/den Staubprobe(n) vorliegen innenräumlichen Ursprungs ist/sind, durch Schlussfolgern, auf der Basis des quantifizierten Niveaus von Pilzen und/oder Bakterien in der/den Staubprobe(n) in Schritt b und von Hintergrundniveaus für Pilze und/oder Bakterien im Vergleich mit der Zeitspanne, während der der Staub sich gesetzt hat, wie Schritt a entsprechend bestimmt, ob das Niveau von Pilzen und/oder Bakterien dem während einer Zeitspanne sich abgesetztem Staub zuzuschreiben ist oder einem Innenraumbefall durch die festgestellten Pilze und/oder Bakterien zuzuschreiben ist,
wobei die Abwesenheit einer oder mehrerer jahreszeitlicher Pollenspezies in der/den Proben(n), in Schritt a bestimmt, nachweist, dass der Staub längstens während einer Zeitspanne seit der Jahreszeit abgesetzt gewesen ist, während der die abwesende eine oder mehrere jahreszeitliche Pollenspezies in der Luft im Freien vorlag und/oder
wobei das Feststellen von jahreszeitlichen Pollenspezies in der Probe/den Proben, in Schritt a bestimmt, nachweist, dass der Staub während einer Zeitspanne abgesetzt gewesen ist, die mindestens zu der Jahreszeit begann, als die eine oder mehreren jahreszeitlichen Pollenspezies in der Luft im Freien vorlag/vorlagen.

2. Verfahren nach Anspruch 1, wobei der Schritt c durch Bestimmen ausgeführt wird, ob das Vorliegen und wahlweise das Niveau jahreszeitlicher Pollenspezies, die in Schritt a) festgestellt worden ist, mit den Pollen und den Pilzen und/oder Bakterien unverträglich ist, die gleichzeitig in die Staubprobe von einer Quelle im Freien eingeführt worden sind.

3. Verfahren nach einem der Ansprüche 1-2, wobei das Niveau des einen oder der mehreren jahreszeitlichen Pollen in Schritt a quantifiziert wird.

4. Verfahren nach einem der Ansprüche 1-3, wobei die qualitative Feststellung und Quantifizierung des Niveaus der einen oder mehreren jahreszeitlichen Pollenspezies in Schritt a unter Anwendung einer Nukleotidsequenz ausgeführt wird, die für jede Spezies oder Gruppe von Spezies von Pollen spezifisch ist.

5. Verfahren nach einem der Ansprüche 1-4, wobei die eine oder mehreren jahreszeitlichen Pollenspezies aus der Gruppe ausgewählt ist bestehend aus:
*Alnus*/*corylus spp., Betula spp., Poaceae spp.* und *Artemisia spp.*

6. Verfahren nach einem der Ansprüche 1-5, wobei die Feststellung und wahlweise Quantifizierung einer oder mehrerer jahreszeitlicher Pollenspezies in Schritt a und die Feststellung und Quantifizierung der Pilze und/oder Bakterien in Schritt b an derselben Probe und im selben Arbeitsgang ausgeführt werden.

7. Verfahren nach einem der Ansprüche 1-6, wobei die Pilze und/oder Bakterien aus der Gruppe ausgewählt sind bestehend aus:
*Absidia spp.*
*Acremonium spp.*
*Alternaria spp.*
*Aspergillus spp.*
*Aureobasidium spp.*
*Chaetomium spp.*
*Chrysonilia spp.*
*Cladosporium spp.*
*Curvularia spp.*
*Emericella spp.*
*Epicoccum spp.*
*Eurotium spp.*
*Fusarium spp.*
*Geomyces spp.*
*Geotrichum spp.*
*Gliocladium spp.*
*Gliomastix spp.*
*Memnoniella spp.*
*Mucor spp.*
*Myrothecium spp.*
*Oidiodendron spp.*
*Paecilomyces spp.*
*Penicillium spp.*
*Phialophora spp.*
*Phoma spp.*
*Rhizopus stolonifer*
*Scopulariopsis spp.*
*Sistotrema spp.*
*Stachybotyris spp.*
*Streptomyces spp.*
*Trichoderma spp.*
*Ulocladium spp.*
*Wallemia spp.*

8. Verfahren nach Anspruch 7, wobei die Pilze und/oder Bakterien aus der Gruppe ausgewählt sind bestehend aus:
*Acremonium strictum*
*Alternaria alternata*
*Aspergillus fumigatus*
*Aspergillus glaucus grp.*
*Aspergillus niger*
*Aspergillus versicolor*
*Chaetomium globosum*
*Cladosporium cladosporides*
*Cladosporium herbarum*
*Cladosporium sphaerospermum*
*Mucor*/*Rhizop grp*
*Penicillium*/*Aspergillus*/*Paecilomyces spp.*
*Penicillium chrysogenum*
*Penicillium expansum*
*Rhizopus stolonifer*
*Stachybotyris chartarum*
*Streptomyces spp.*
*Trichoderma viride*
*Ulocladium chartarum*
*Wallemia sebi*

9. Verfahren nach einem der Ansprüche 1-8, ferner den Schritt antimykotischer und/oder antibakterieller Behandlung des Innenbereichs umfassend, wenn der Ursprung von Pilzen und/oder Bakterien in der Staubprobe von Innenraumursprung ist.

10. Verwendung qualitativer Feststellung von jahreszeitlichen Pollen bei der Bestimmung des Ursprungs von Pilzen und/oder Bakterien aus einer Innenraumstaubprobe durch ein Verfahren wie in einem der Ansprüche 1-9 definiert.

## Revendications

1. Procédé de détermination de l'origine de champignons et/ou de bactéries dans un ou plusieurs échantillons de poussières provenant d'une zone intérieure, le procédé comprenant les étapes consistant à :
a) détecter de manière qualitative au moins une espèce de pollen saisonnière présente dans le ou les échantillons de poussières,
b) détecter et quantifier le niveau de champignons et/de bactéries dans le ou les échantillons de poussières, et
c) utiliser l'identité de l'une ou plusieurs espèces de pollen saisonnières afin de déterminer si les champignons et/ou les bactéries présents dans le ou les échantillons de poussières sont d'origine intérieure en concluant en fonction du niveau quantifié de champignons et/ou bactéries dans le ou les échantillons de poussières dans l'étape b et les niveaux de fond pour les champignons et/ou les bactéries par rapport à la période de temps pendant laquelle la poussière est retombée telle que déterminée conformément à l'étape a, si le niveau de champignons et/ou de bactéries provient de la poussière retombée pendant une période de temps ou provient de l'infestation intérieure des champignons détectés et/ou des bactéries détectées,
dans lequel l'absence d'une ou de plusieurs espèces de pollen saisonnières dans le ou lesdits échantillons déterminés dans l'étape a permet d'établir que la poussière est retombée pendant une période de temps la plus longue depuis le moment de l'année où ladite au moins une espèce de pollen saisonnière absente était présente dans l'air extérieur et/ou
dans lequel la détection d'espèces de pollen saisonnières dans le ou lesdits échantillons déterminés dans l'étape a permet d'établir que la poussière est retombée pendant une période de temps commençant au moins depuis le moment de l'année où ladite au moins une espèce de pollen saisonnière est présente dans l'air extérieur.

2. Procédé selon la revendication 1, dans lequel l'étape c est réalisée en déterminant si la présence et éventuellement le niveau d'espèces de pollen saisonnières détecté dans l'étape a) est incompatible avec le pollen et les champignons et/ou les bactéries ayant été introduits de manière simultanée dans l'échantillon de poussières provenant d'une source extérieure.

3. Procédé selon l'une quelconque des revendications 1 à 2, dans lequel le niveau de l'un ou de plusieurs pollens saisonniers est quantifié dans l'étape a.

4. Procédé selon l'une quelconque des revendications 1 à 3, dans lequel la détection qualitative et la quantification du niveau de l'une ou de plusieurs espèces de pollen saisonnières dans l'étape a est réalisée en utilisant une séquence de nucléotides spécifique de chaque espèce ou groupe d'espèces de pollen.

5. Procédé selon l'une quelconque des revendications 1 à 4, dans lequel l'une ou plusieurs espèces de pollen saisonnières sont choisies dans le groupe constitué de :
*Alnusjcorylus spp., Betula spp., Poaceae spp. et Artemisia spp.*

6. Procédé selon l'une quelconque des revendications 1 à 5, dans lequel la détection et la quantification éventuelle de l'une ou de plusieurs espèces de pollen saisonnières dans l'étape a et la détection et la quantification des champignons et/ou des bactéries dans l'étape b est réalisée sur le même échantillon et lors de la même opération.

7. Procédé selon l'une quelconque des revendications 1 à 6, dans lequel les champignons et/ou les bactéries sont choisis dans le groupe constitué de :
*Absidia spp.*
*Acremonium spp*
*Alternaria spp.*
*Aspergillus spp.*
*Aureobasidium spp.*
*Chaetomium spp.*
*Chrysonilia spp.*
*Cladosporium spp.*
*Curvularia spp.*
*Emericella spp.*
*Epicoccum spp.*
*Eurotium spp.*
*Fusarium spp.*
*Geomyces spp.*
*Geotrichum spp.*
*Gliocladium spp.*
*Gliomastix spp.*
*Memnoniella spp.*
*Mucor spp.*
*Myrothecium spp.*
*Oidiodendron spp.*
*Paecilomyces spp.*
*Penicillium spp.*
*Phialophora spp.*
*Phoma spp.*
*Rhizopus stolonifer*
*Scopulariopsis spp.*
*Sistotrema spp.*
*Stachybotrys spp.*
*Streptomyces spp.*
*Trichoderma spp.*
*Ulocladium spp.*
*Wallemia spp.*

8. Procédé selon la revendication, dans lequel les champignons et/ou les bactéries sont choisis dans le groupe constitué de :
*Acremonium strictum*
*Alternaria alternata*
*Aspergillus fumigatus*
*Aspergillus glaucus grp.*
*Aspergillus niger*
*Aspergillus versicolor*
*Chaetomium globosum*
*Cladosporium cladosporides*
*Cladosporium herbarum*
*Cladosporium sphaerospermum*
*Mucor* / *rhizop. grp*
*Penicillium* / *Aspergillus* / *Paecilomyces spp.*
*Penicillium chrysogenum*
*Penicillium expansum*
*Rhizopus stolonifer*
*Stachybotrys chartarum*
*Streptomyces spp.*
*Tricoderma viride*
*Ulocladium chartarum*
*Wallemia sebi.*

9. Procédé selon l'une quelconque des revendications 1 à 8 comprenant en outre l'étape de traitement antifongique et/ou antibactérien de la zone intérieure si l'origine des champignons et/ou des bactéries dans l'échantillon de poussières est d'origine intérieure.

10. Utilisation de la détection qualitative de pollen saisonniers dans la détermination de l'origine de champignons et/ou de bactéries provenant d'un échantillon de poussières intérieur au moyen d'un procédé tel que défini dans l'une quelconque des revendications 1 à 9.
